(19) Europäisches Patentamt
European Patent Office
Office européen des brevets

(11) **EP 2 318 821 B1**

(12) **EUROPÄISCHE PATENTSCHRIFT**

(45) Veröffentlichungstag und Bekanntmachung des Hinweises auf die Patenterteilung:
**23.11.2011 Patentblatt 2011/47**

(21) Anmeldenummer: **09796290.6**

(22) Anmeldetag: **03.09.2009**

(51) Int Cl.:
*G01N 21/64* (2006.01)  *G01N 33/58* (2006.01)

(86) Internationale Anmeldenummer:
**PCT/DE2009/001249**

(87) Internationale Veröffentlichungsnummer:
**WO 2010/025715 (11.03.2010 Gazette 2010/10)**

(54) **VERFAHREN ZUR EXAKTEN BESTIMMUNG DER FLUORESZENZ IN EINEM SCHICHTSYSTEM, BEISPIELSWEISE DEM AUGE**

METHOD FOR PRECISELY DETERMINING THE FLUORESCENCE IN A LAYER SYSTEM, SUCH AS THE EYE

PROCÉDÉ PERMETTANT LA DÉTERMINATION EXACTE DE LA FLUORESCENCE DANS UN SYSTÈME STRATIFIÉ, TEL QUE L OEIL PAR EXEMPLE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **03.09.2008 DE 102008045886**

(43) Veröffentlichungstag der Anmeldung:
**11.05.2011 Patentblatt 2011/19**

(73) Patentinhaber: **Heidelberg Engineering GmbH**
**69121 Heidelberg (DE)**

(72) Erfinder: **SCHWEITZER, Dietrich**
**07806 Neustadt/Orla (DE)**

(74) Vertreter: **Donath, Dirk et al**
**Patent- und Rechtsanwälte**
**Bock, Bieber, Donath**
**Hans-Knöll-Straße 1**
**07745 Jena (DE)**

(56) Entgegenhaltungen:
**WO-A2-2006/009910**

• **SCHWEITZER D ET AL: "Method for simultaneous detection of functionality and tomography" PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, Bd. 7368, 2009, Seiten 736804-1-736804-9, XP002568108 ISSN: 0277-786X**

• **MYCEK M -A ET AL: "Simulations of time-resolved fluorescence in multilayered biological tissues: applications to clinical data modeling" PROCEEDINGS OF THE SPIE - THE INTERNATIONAL SOCIETY FOR OPTICAL ENGINEERING SPIE-INT. SOC. OPT. ENG USA, Bd. 4958, 2003, Seiten 51-59, XP002568109 ISSN: 0277-786X**

• **VISHWANATH K ET AL: "A Monte-Carlo model for time-resolved fluorescence from a two-layered turbid medium" OSA TRENDS IN OPTICS AND PHOTONICS, WASHINGTON, DC, US, Bd. 88, 1. Januar 2003 (2003-01-01), Seiten 832-834, XP009129341 ISSN: 1094-5695**

• **PFEFER T J ET AL: "Computational modeling of device-tissue interface geometries for time-resolved fluorescence in layered tissue" PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, Bd. 6083, Nr. 1, 9. Februar 2006 (2006-02-09), Seiten 1-9, XP002568110 ISSN: 0277-786X**

• **SCHWEITZER D ET AL: "In vivo measurement of time-resolved autofluorescence at the human fundus" JOURNAL OF BIOMEDICAL OPTICS NOVEMBER/DECEMBER 2004 SPIE US, Bd. 9, Nr. 6, November 2004 (2004-11), Seiten 1214-1222, XP002568111**

- SCHWEITZER D ET AL: "Comparison of time-resolved autofluorescence in the eye-ground of healthy subjects and patients suffering from age-related macular degeneration" PROCEEDINGS OF THE SPIE - THE INTERNATIONAL SOCIETY FOR OPTICAL ENGINEERING SPIE-INT. SOC. OPT. ENG USA, Bd. 5862, Nr. 1, 2005, Seiten 58620R-1-58620R-12, XP002568112 ISSN: 0277-786X

**Beschreibung**

Die Erfindung betrifft ein Verfahren zur exakten Bestimmung der Fluoreszenz in einem Schichtsystem, beispielsweise dem Auge.

Das Verfahren kann zur Untersuchung des schichtspezifischen Fluoreszenzverhaltens endogener oder exogener Fluorophore in Organen biologischer Objekte wie dem Auge, der Haut, dem Darm, der Blase usw. eingesetzt werden. Mit der Erfindung kann die Diffusion markierter Pharmaka durch morphologische Strukturen sowie deren Anlagerung an Zielstrukturen kontrolliert werden. Sie kann ebenfalls zur Untersuchung der Fluoreszenz in geschichteten pflanzlichen Strukturen angewendet werden.

Mit der Erfindung wird eine tomographische Rekonstruktion eines Objektes mit einer Struktur aus fluoreszierenden Schichten erreicht. Auch eine Fertigungskontrolle bei der Herstellung von Produkten mit geschichteter Struktur ist möglich.

Werden Schichtsysteme zur Fluoreszenz angeregt, so emittieren jeweils in den einzelnen Schichten schichtstrukturspezifisch lokale Fluorophore, wobei zu deren Fluoreszenzauswertung bekannter Weise das summarische Abklingverhalten der Summenfluoreszenz untersucht wird. Insbesondere beim menschlichen Auge werden Fluorophore in den anatomischen Schichten des vorderen Auges sowie in den Schichten des Augenhintergrundes angeregt. Sowohl die Fluoreszenz endogener Fluorophore als auch fluoreszierende Marker können zur Diagnostik z. B. für die Beurteilung des Stoffwechselzustandes herangezogen werden. Die Fluoreszenzauswertung des Auges liefert somit Informationen, die für die ophthalmologische Untersuchung und den Zustand des Auges sowie zur Bewertung eventuelle Schädigungen und/oder Früherkennung von Erkrankungen des Auges bzw. seiner Bestandteile sehr wichtig sein können. Diese Informationen betreffen die Auswertung des Abklingverhaltens der vom gesamten Objekt, beispielsweise des Auges, gewonnenen substanzspezifischen funktionellen Informationen. Es besteht weiterhin die bisher ungelöste Forderung, Informationen über den schichtspezifischen Entstehungsort der substanzspezifischen Fluoreszenz zu erhalten.

Außerdem wäre es zweckmäßig, wenn solche Messungen und Auswertungen auch unmittelbar in der klinischen Routine ophthalmologischer Untersuchungen möglichst aufwandgering durchgeführt werden könnten und dabei hochgenaue Untersuchungsergebnisse geliefert würden.

Die Erfindung ist jedoch nicht auf Anwendungen bezüglich des Auges als Schichtsystem beschränkt.

Bei Messungen der Fluoreszenz in Mischungen von Fluorophoren wird im Allgemeinen davon ausgegangen, dass sich die zu untersuchenden Substanzen in der gleichen Objektebene befinden. Das gilt sowohl für die Untersuchung von endogenen Fluorophoren als auch von fluoreszierenden Markern in Zell- oder Gewebekulturen.

Mittels 2-oder Multi-Photonenanregung (K. König, I. Riemann: High-resolution multiphoton tomography of human skin with subcellular spatial resolution and picosecond time resolution. Journal of Biomedical Optics 8(3), 2003, 432-439) können einzelne Punkte einer Schichten eines Objekts mit hoher geometrischer Auflösung zur Fluoreszenz angeregt werden. In Verbindung mit Scanning Systemen (Scanner Mikroskope) kann die Fluoreszenz einer ganzen Schicht bestimmt werden (W. Denk, J. H. Strickler, W. W. Webb: Two-photon laser scanning microscopy, Science 248, 1990, 73-76; B. R. Masters, P. T. C. So, E. Gratton: Multiphoton excitation fluorescence microscopy of in vivo human skin. Ann. N.Y. Acad. Sci. 838, 1998, 58-67). Nach Fokussieren auf weitere Schichten kann im Prinzip die geometrische Struktur der fluoreszierenden Schichten eines Objekts bestimmt werden.

Um die für 2-oder Multi-Photonenanregung erforderliche Energiedichte in Fokus des Anregungssystems zu erreichen, sind bei hohen Strahlungsleistungen optische Systeme mit hoher numerischer Apertur erforderlich. Hierfür sind Immersionsobjektive von Mikroskopen geeignet. Damit können fluoreszierende Schichten eines mikroskopischen Präparates oder der Haut bis zu einer Schichtdicke von ca. 1 mm in einem kleinen Feld untersucht werden.

Bei stark absorbierenden Strukturen, wie dem retinalen Pigmentepithel, tritt bereits eine Schädigung auf, wenn die applizierte Strahlungsenergie lediglich um den Faktor 3 größer ist als die Energie zum Anregen der Fluoreszenz. Die durch die geweitete Iris und die Brennweite des Auges bestimmte geringe Apertur des Auges schließt die Anwendung von 2-oder Multiphotonenprozessen zur Untersuchung der Fluoreszenz des Augenhintergrundes am lebenden Auge ebenfalls aus.

Die gleichzeitige Bestimmung der Fluoreszenz verschiedener Schichten eines Objekts ist mit 2-oder Multi-Photonenanregung prinzipiell nicht möglich, da die Fokussierung mit hoher Genauigkeit nur in eine Fokalebene möglich ist. Damit ist die gleichzeitige Messung der Fluoreszenz verschiedener Schichten des Auges, beispielsweise des vorderen und des hinteren Augenabschnittes, grundsätzlich mit 2-oder Multi-Photonenanregung nicht möglich.

Die Untersuchung der Autofluoreszenz oder der Fluoreszenz exogener Marker kann am Auge mittels Funduskameras oder Laser Scanner Ophthalmoskopen vorgenommen werden. Eingeführter Stand der Technik ist die Messung der statischen Fluoreszenz, vorwiegend des Augenhintergrundes (A. von Rückmann, F. W. Fitzke, A. C. Bird: Distribution of fundus autofluorescence with a scanning laser ophthalmoscope, Br. J Ophthalmol 79, 1995, 407-412; F. G. Holz et al.: Fundus autofluorescence and development of geographic atrophy in agerelated macular degeneration. Invest Ophthalmol Vis Sci 42, 2001, 1051-1056).

Durch Kombination eines modifizierten Ophthalmoskops mit einem Spektrographen kann das Fluoreszenzspektrum eines ausgewählten Bereiches am Augenhintergrund berechnet werden (F. C. Delori et al.: In vivo fluorescence of the ocular fundus exhibits retinal pigment epithelium lipofuscin characteristics. Invest Ophthalmol Vis Sci 36, 1995, 718- 729; D. Schweitzer et al.: Die altersabhängige Makulopathie - Vergleichende Untersuchungen zwischen Patienten, deren Kindern und Augengesunden. Ophthalmologe 97, 2000, 84-90).

In neueren Entwicklungen wird die dynamische Fluoreszenz des Auges nach Anregung mit ps-Laserpulsen gemessen (D. Schweitzer et al.: In vivo measurement of time-resolved autofluorescence at the human fundus. J Biomed Opt 9, 2004, 1214 - 1222; D. Schweitzer et al.: Towards metabolic mapping of the human retina. Microscopy Research and Technique 70, 2007, 410-419). Dabei wird zur Auswertung der dynamischen (zeitaufgelösten) Fluoreszenz des Auges (D. Schweitzer et al.: In vivo measurement of time-resolved autofluorescence at the human fundus, J Biomed Opt 9, 2004, 1214-1222) oder anderer Objekte davon ausgegangen, dass die Fluoreszenz aller Fluorophore in der gleichen Fokalebene entsteht. Zur Approximation des summarischen Abklingverhaltens der Fluoreszenz des Auges als Schichtsystem wird beispielsweise eine multiexponentielle Modellfunktion entsprechend Gleichung (1) verwendet:

$$\frac{I(t)}{I_0} = \sum_{j=1}^{p} \alpha_i \cdot e^{-\frac{t}{\tau_i}} + b \qquad (1)$$

mit
$I_o$: maximale Fluoreszenzintensität
$I(t)$: Fluoreszenz zum Zeitpunkt $t$
$\tau_i$: Abklingzeit der Komponente $i$
$\alpha_i$: präexponentieller Faktor $i$
$b$: Untergrundintensität.

Da in dieser Modellfunktion angenommen wird, dass alle Fluorophore in nur einer Schicht lokalisiert sind, besteht keine Möglichkeit, auf die Entstehungsorte der einzelnen Fluoreszenzen zu schließen. Damit ist die Auswertung der zeitabhängigen Summenfluoreszenz eines Objekts, das verschiedene fluoreszierende Schichten enthält, näherungsweise möglich, aber unsicher und ungenau.

WO-2006 009 910 A1 (Absätze 71, 109-111) offenbart ein Verfahren zur Untersuchung und Diagnose von Hautschichten des menschlichen Körpers mittels zeitaufgelösten Fluoreszenz. Gemäß den Absätzen 109-110 offenbart dieses Dokument eine Bestimmung der Ortung der Streuung und Absorption durch eine Verarbeitung der zeitabhängigen Signale von einem Detektor mittels standardmäßigen umgekehrten Algorithmen. Diese Inversion umfasst eine iterative Bestimmung der Eigenschaften des zu untersuchenden Materials, die der Unterschied zwischen der gemessenen und der berechneten Streuung bzw. Absorption minimiert. Diese Rechung erfolgt mittels eines Algorithmus durch mathematische Lösung einer Gleichung, die der physikalische Verlauf von elektromagnetischen Wellen durch das Medium darstellt. Gemäß diesem Algorithmus wird die Reflexion in zeitlich fortschreitenden Schritte gemessen und die inkrementelle Tiefe der Lichtpenetration berechnet. Die optischen Eigenschaften dieser inkrementellen Tiefe wird berechnet anhand von den Materialeigenschaften bis zu diesen Tiefe unter Kenntnis der Reflexion zu diesem Zeitpunkt. Diese Prozedur wird dann wiederholt bis die Streuung bzw. Absorption des Mediums bei jedem Ort festgestellt wird. Gemäß dem folgenden Absatz 111 liefert eine mathematische Analyse des zeitabhängigen Signals Information bezüglich der Struktur und Physiologie der Haut wobei die Reflexion-, bzw. die Transmission- bzw. die Fluoreszenzsignale Maxima, Minima, Scheitelpunkte und Beugungspunkte aufweisen, die weitere Information bezüglich der Haustruktur darstellen.

Der Erfindung liegt die Aufgabe zugrunde, ein möglichst einfaches und aufwandgeringes Verfahren zur Auswertung der Fluoreszenz in einem Schichtsystem zu schaffen, mit dem das summarische Abklingverhalten der Fluoreszenz sehr exakt ausgewertet werden kann und mit dem gleichzeitig auf die Entstehungsorte der einzelnen Fluoreszenzen des Schichtsystems geschlossen werden kann.

Erfindungsgemäß werden zur Auswertung des summarischen Abklingverhaltens der zeitaufgelösten Fluoreszenz des Schichtsystems jeweils die Entstehungszeitpunkte der Fluoreszenzen in den einzelnen Schichten des Schichtsystems bestimmt, indem für die betreffenden Fluoreszenzen jeweils schichtspezifische zeitabhängige Parameter in der Modellfunktion zur Approximation an den gemessenen Verlauf des summarischen Abklingverhaltens berücksichtigt werden, die jeweils den -zeitlichen Beginn der Fluoreszenz in der betreffenden Schicht angeben. Auf diese Weise kann das summarische Abklingverhalten der zeitaufgelösten Fluoreszenz des Schichtsystems unter Berücksichtigung der Entstehungszeitpunkte in den einzelnen Schichten des Schichtsystems exakter ausgewertet werden.

Die schichtspezifischen zeitabhängigen Parameter können beispielsweise jeweils als Fitparameter $tc_i$ in die besagte Modellfunktion eingebracht werden. Ein Beispiel für einen multiexponentiellen Fit zeigt die allgemeine Formel (2)

$$\frac{I(t)}{I_0} = \sum_{i=1}^{p} \alpha_i \cdot e^{-\frac{t-tc_i}{\tau_i}} + b \quad \text{(2)} \quad \text{mit} \quad e^{-\frac{t-tc_i}{\tau_i}} = 0 \quad \text{für } t < tc_i$$

und

$I_o$:     maximale Fluoreszenzintensität
I(t):     Fluoreszenz zum Zeitpunkt t
$\alpha_i$:     präexponentieller Faktor i
$\tau_i$:     Abklingzeit der Komponente i in Schicht i
$tc_i$:     Zeitpunkt der Entstehung der Fluoreszenz in Schicht i
b:     Untergrundintensität.

Gleichzeitig kann aus diesen gewonnenen schichtspezifischen zeitabhängigen Parametern, welche die Entstehungszeitpunkte der einzelnen Fluoreszenzen des Schichtsystems repräsentieren, vorteilhaft der lokale Abstand der Entstehungsorte der Fluoreszenzen berechnet werden, indem jeweils die Differenz dieser Parameter mit der Lichtgeschwindigkeit in der betreffenden Schicht multipliziert wird. Aus diesen lokalen Abständen lassen sich die auf einfachste Weise die absoluten Entstehungsorte der Fluoreszenzen berechnen. Mit der Erfindung kann somit zusätzlich zur oben beschriebenen exakteren Auswertung des summarischen Abklingverhaltens der zeitaufgelösten Fluoreszenz des Schichtsystems auch gleichzeitig der Entstehungsort der Fluoreszenz in der geometrische Struktur des Schichtsystem ermittelt werden, was bisher in der Fachwelt ein ungelöstes Problem darstellte.

Mit der Erfindung kann unterschieden werden, ob in einer Schicht multiexponentielles Fluoreszenz Abklingverhalten vorliegt oder ob ein summarisches multiexponentielles Abklingverhalten durch das Abklingverhalten von Fluorophoren in einzelnen Schichten entstanden ist.

Bei Anwendungen in der Augenheilkunde können mindestens die Fluoreszenzen der Augenlinse, der Netzhaut und des retinalen Pigmentepithels von einander unterschieden werden. Da mittels der zeitaufgelösten Autofluoreszenz möglichst in Verbindung mit veränderbarer spektraler Anregung und Detektion der zeitaufgelösten Fluoreszenz in verschiedenen Spektralbreichen Aussagen über den zellulären Stoffwechselzustand gewonnen werden können, ist es möglich, mit Hilfe der Erfindung Änderungen des Stoffwechselzustandes in einzelnen funktionellen Schichten des Auges oder anderer optisch zugänglicher Organe zu bestimmen.

Die Erfindung ist nicht auf die Detektion der Autofluoreszenz in einzelnen Schichten, insbesondere die Auswertung von summarisch gewonnenem Abklingverhalten der Autofluoreszenz, beschränkt. Sie kann in gleicher Weise für die Auswertung der zeitabhängigen Fluoreszenz von externen Markern in Schichtsystemen auch in Verbindung mit endogenen Fluorophoren angewendet werden. Beispielsweise kann untersucht werden, in welcher Schicht des Objekts sich eine fluoreszierende Markersubstanz anlagert. Ebenfalls ist es möglich, den örtlichen Verlauf der Diffusion eines topisch applizierten fluoreszierenden Markers in Abhängigkeit von der Zeit zu bestimmen. So kann die örtliche Änderung eines fluoreszierenden Medikamentes oder eines mit Markern versehenen Medikamentes in Abhängigkeit von der Zeit, beispielsweise bei Diffusionsprozessen topisch applizierter Pharmaka durch den Glaskörper des Auges, bestimmt werden, was bei pharmako-kinetischen Untersuchungen von Interesse ist.

Da sich die Abklingzeit eines Fluorophors ändert, wenn sich dieses beispielsweise an ein Protein bindet, kann mit der Erfindung auch die Reaktion des Medikaments in der entsprechenden anatomischen Schicht beurteilt werden.

Die Erfindung soll nachstehend anhand eines in der Zeichnung dargestellten Ausführungsbeispiels näher erläutert werden.

Es zeigen:

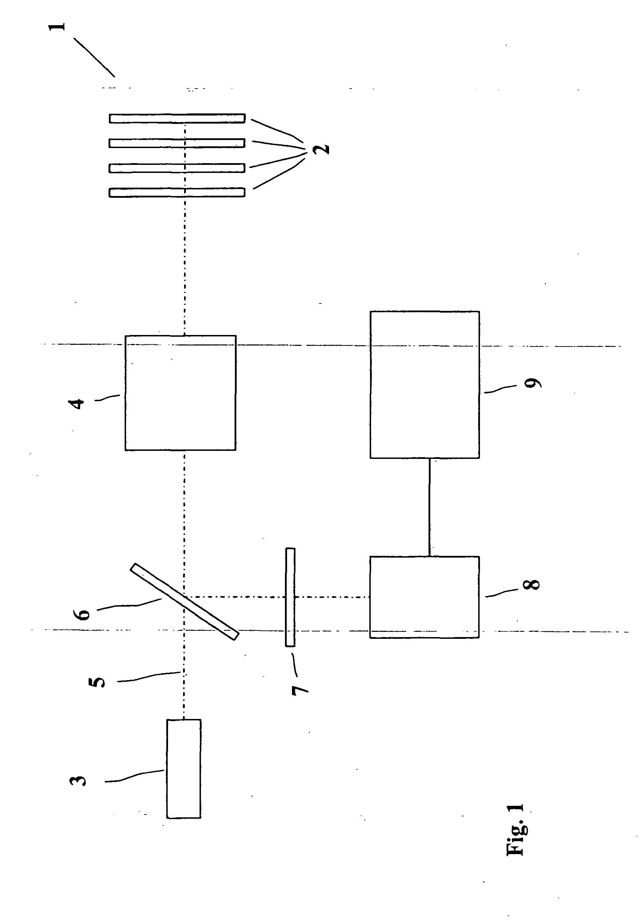

Fig. 1:     Schematischer Aufbau zum Messen der zeitaufgelösten Fluoreszenz eines Objekts aus fluoreszierenden Schichten

Fig. 2:     Schematische Darstellung des summarischen Zeitverlaufs der Fluoreszenz eines Objekts aus diesen fluoreszierenden Schichten

Mit einem Laser Scanner Ophthalmoskop werden alle Orte eines in der Fluoreszenz auszuwertenden Feldes eines Objekts 1 (beispielsweise einem Auge), bestehend aus fluoreszierenden Schichten 2, durch Beleuchtung mit einem Pulslaser 3 während des spektrometrischen Abtastens durch ein Scanner System 4 seriell zur Fluoreszenz angeregt. Dabei werden alle Schichten 2 längs der Ausbreitungsrichtung eines Anregungsstrahls 5 des Pulslasers 3 jeweils durch denselben Anregungsstrahl 5, der über einen dichroitischen Spiegel 6 und das Scanner System 4 auf das Objekt 1 trifft, zur Fluoreszenz angeregt. Das durch diese Fluoreszenzanregung entstehende und vom Scanner System 4 erfasste Fluoreszenzlicht wird vom dichroitischen Spiegel 6 auf einen Blockfilter 7 gelenkt, durch den das besagte Fluoreszenzlicht vom Anregungslicht getrennt wird. Nach dieser Trennung wird das zeitaufgelöste Fluoreszenzlicht in einer Einheit 8 zur zeitaufgelösten Detektion des Fluoreszenzlichtes summarisch von allen fluoreszierenden Schichten 2 des Objekts 1 (beispielsweise mittels des an sich bekannten zeitkorrelierten Einzelphotonenzählens) detektiert.

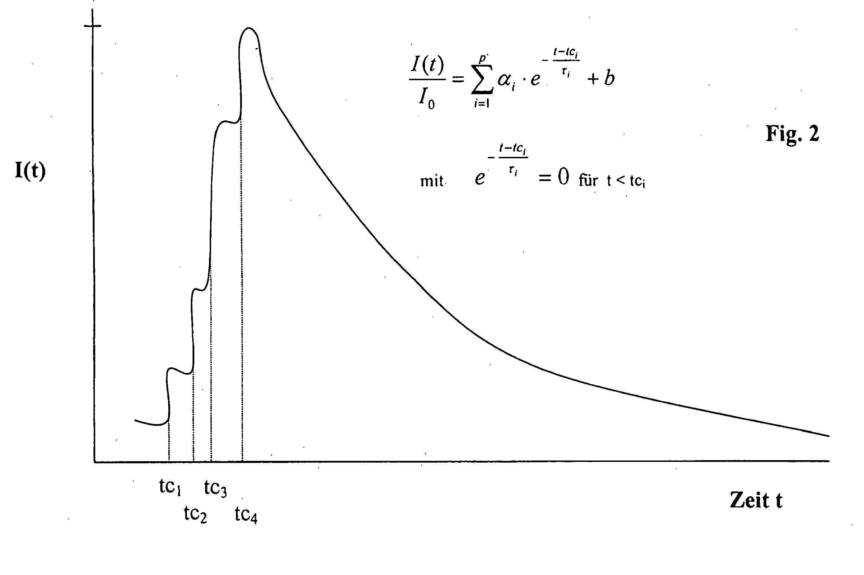

Mit der Erfindung wird der Anstieg der Fluoreszenzintensität als stufiger Verlauf infolge der schichtspezifischen Fluororeszenz detailliert ausgewertet, wobei die vier Stufen in der ansteigenden Fluoreszenzintensität von Fig. 2 die in den vier fluoreszierenden Schichten 2 des Objekts 1 jeweils infolge der Anregung entstehenden schichtspezifische Fluoreszenz widerspiegeln.

Erfindungsgemäß wird dieser stufige Anstiegsverlauf der Fluoreszenzintensität analysiert, indem für die betreffenden Fluorophore der Schichten 2 des Objekts 1 jeweils die Entstehungszeitpunkte bestimmt und zeitlich zueinander in Relation gebracht werden.

Zur Auswertung der summarischen Fluoreszenz wird bekannter Weise das Abklingverhalten der Fluoreszenz anhand einer Modellfunktion in einem Rechner 9 ermittelt. Erfindungsgemäß werden dabei für eine exakte Auswertung die vorgenannten Entstehungszeitpunkte der einzelnen schichtrelevanten Fluorophore als Parameter in der besagten Modell funktion berücksichtigt. Im Ausführungsbeispiel wird deshalb für eine multiexponentielle Modellfunktion zur Approximation des zeitlichen Abfalls der Fluoreszenz für jede Komponente zusätzlich zu den zu optimierenden Parametern Lebensdauer $\tau_i$ und präexponentieller Faktor $\alpha_i$ ein weiterer zu optimierender Faktor $tc_i$ eingeführt, der jeweils den Entstehungszeitpunkt der Fluoreszenz der Komponente i kennzeichnet.

$$\frac{I(t)}{I_0} = \sum_{i=1}^{p} \alpha_i \cdot e^{-\frac{t-tc_i}{\tau_i}} + b \qquad (2) \qquad \text{mit} \quad e^{-\frac{t-tc_i}{\tau_i}} = 0 \text{ für } t < tc_i$$

und

$I_o$:  maximale Fluoreszenzintensität
$I(t)$:  Fluoreszenz zum Zeitpunkt t
$\alpha_i$:  Präexponentieller Faktor i
$\tau_i$  Abklingzeit der Komponente i in Schicht i
$tc_i$:  Zeitpunkt der Entstehung der Fluoreszenz in Schicht i
b:  Untergrundintensität.

Mit dieser erweiterten Modellfunktion wird eine zuverlässige und exakte Auswertung eines summarisch gemessenen Fluoreszenzverlaufes im Objekt 1 aus den fluoreszierenden Schichten 2 erreicht. Die Differenzen der somit bestimmten Werte $tc_i$ (im Ausführungsbeispiel $tc_1$, $tc_2$, $tc_3$ und $tc_4$) geben den zeitlichen Unterschied in der Entstehung der Fluoreszenz in den einzelnen Schichten 2 an. Werden für mehrere Komponenten gleiche Werte $tc_i$ bestimmt, so liegt in der entsprechenden Schicht multiexponentielles Abklingverhalten vor.

Werden die Werte $tc_i$ jeweils mit der Lichtgeschwindigkeit in der betreffenden Schicht 2 des Objekts 1 multipliziert, so lassen sich aus diesem Produkt die geometrischen Abstände zwischen den fluoreszierenden Schichten 2 berechnen, so dass Rückschlüsse auf die Fluoreszenzen in den einzelnen Schichten 2 des Objekts 1 möglich sind. Diese Rückschlüsse sind beispielsweise für ophthalmologische Untersuchungen des Auges als Objekt 1 bedeutsam.

Wird die Formel (2) für die Auswertung des Abklingverhaltens der summarischen Fluoreszenz an jedem Bildpunkt angewendet, so können aus den Werten $tc_i$ die Lage und die Abstände zwischen den einzelnen Schichten 2 des Objekts 1 und damit die geometrische Struktur der Entstehungsorte der einzelnen Fluorophore berechnet werden.

Damit wird durch die Erfindung nicht nur eine genaue Auswertung des Abklingverhaltens der summarischen Fluoreszenz eines Objekts aus fluoreszierenden Schichten erreicht, sondern es gelingt durch die Erfindung, die Verbindung zwischen der zeitaufgelösten Messung der Autofluoreszenz zur Beurteilung insbesondere des Stoffwechselstatus im Auge mit der geometrischen Anordnung der fluoreszierenden Schichten als Äquivalent zur optischen Kohärenztomographie herzustellen.

Bezugszeichenliste

1    - Objekt aus fluoreszierenden Schichten 2

2    - fluoreszierende Schicht

3    - Pulslaser

4    - Scanner System

5    - Anregungsstrahl

6    - dichroitischer Spiegel

7    - Blockfilter

8    - Einheit zur zeitaufgelösten Detektion des Fluoreszenzlichtes

9    Rechner zur Auswertung der summarischen zeitaufgelösten Fluoreszenz $tc_1$, $tc_2$, $tc_3$, $tc_4$ Entstehungszeitpunkte der Fluoreszenzen in den Schichten 2

**Patentansprüche**

1. Verfahren zur exakten Bestimmung der zeitaufgelösten Fluoreszenz in einem Schichtsystem (1), beispielsweise dem Auge, wobei das summarische Abklingverhalten der Fluoreszenz des Schichtsystems (1) anhand einer den Messungen anzupassenden Modellfunktion ausgewertet wird, **dadurch gekennzeichnet, dass** zur Auswertung des summarischen Abklingverhaltens der zeitaufgelösten Fluoreszenz des Schichtsystems (1) jeweils die Entstehungszeitpunkte der Fluoreszenzen in den einzelnen Schichten (2) des Schichtsystems (1) bestimmt werden, indem für die betreffenden Fluoreszenzen schichtspezifische zeitabhängige Parameter in der Modellfunktion zur Approximation an den gemessenen Verlauf des summarischen Abklingverhaltens berücksichtigt werden, die jeweils den zeitlichen Beginn der Fluoreszenz in der betreffenden Schicht (2) angeben.

2. Verfahren gemäß Anspruch 1, **dadurch gekennzeichnet, dass** jeweils aus der Differenz der schichtspezifischen zeitabhängigen Parameter durch Multiplikation mit der Lichtgeschwindigkeit in der betreffenden Schicht des Schichtsystems der lokale geometrische Abstand der Entstehungsorte der Fluoreszenzen berechnet wird.

3. Verfahren gemäß Anspruch 2, **dadurch gekennzeichnet, dass** aus den lokalen Abständen der einzelnen Entstehungsorte der Fluoreszenz die geometrischen Strukturen der fluoreszierenden Schichten des Schichtsystems berechnet werden.

4. Verfahren gemäß Anspruch 1, **dadurch gekennzeichnet, dass** die schichtspezifischen zeitabhängigen Parameter, welche die Entstehungszeitpunkte der einzelnen Fluoreszenzen des Schichtsystems repräsentieren, zur Berechnung des summarischen Abklingverhaltens der Fluoreszenz des Schichtsystems in der zur Auswertung herangezogenen Modellfunktion jeweils als Fitparameter $tc_i$ eingeführt werden, beispielsweise für einen multiexponentiellen Fit gemäß allgemeiner Formel (2)

$$\frac{I(t)}{I_0} = \sum_{i=1}^{p} \alpha_i \cdot e^{-\frac{t-tc_i}{\tau_i}} + b \qquad (2)\text{mit} \qquad e^{-\frac{t-tc_i}{\tau_i}} = 0 \ \text{für} \ t < tc_i$$

und

$I_0$: maximale Fluoreszenzintensität
$I(t)$: Fluoreszenz zum Zeitpunkt t
$\alpha_i$: Präexponentieller Faktor i
$\tau_i$: Abklingzeit der Komponente i in Schicht i

$tc_i$: Zeitpunkt der Entstehung der Fluoreszenz in Schicht i

b: Untergrundintensität.

**Claims**

1. Method for precisely determining the time-resolved fluorescence in a layer system (1), for example in the eye, in which the summary decay behavior of the fluorescence of the layer system (1) is evaluated by a model function that has to be adjusted to the measurements, wherein each of the times of origin of the fluorescences in the individual layers (2) of the layer system (1) is determined for analyzing the summary decay behavior of the time-resolved fluorescence of the layer system (1) in that the layer-specific, time-dependent parameters for the corresponding fluorescences, with each of the parameters indicating the time of origin of the fluorescence in the relevant specific layer (2), are considered in the model function for the approximation to the measured course of the summary decay behavior.

2. Method in accordance with claim 1, wherein the local geometric distance of the points of origin of the fluorescences is calculated from the difference of the layer-specific, time-dependent parameters by multiplying it with the light speed in the relevant layer of the layer system.

3. Method in accordance with claim 2, wherein the geometric structures of the fluorescent layers of the layer system are calculated from the local distances of the single points of origin of the fluorescence.

4. Method in accordance with claim 1, wherein the layer-specific, time-dependent parameters, which indicate the times of origin of the individual fluorescences of the layer system, are introduced as fit parameters $tc_i$ in the model function used for the evaluation to calculate the summary decay behavior of the fluorescence in the layer system, for example as a multi-exponential fit according to the general equation (2).

$$\frac{I(t)}{I_0} = \sum_{i=1}^{p} \alpha_i \cdot e^{-\frac{t-tc_i}{\tau_i}} + b \qquad (2) \text{ with } \quad e^{-\frac{t-tc_i}{\tau_i}} = 0 \text{ for } t < tc_i$$

and

$I_o$: maximum fluorescence intensity

$I(t)$: fluorescence at the time t

$\alpha_i$: pre-exponential factor i

$\tau_i$: decay time of the component i in layer i

$tc_i$: time of origin of fluorescence in layer i

b: underground intensity.

**Revendications**

1. Procédé permettant la détermination exacte de la fluorescence à résolution dans le temps dans un système stratifié (1), tel que l'oeil par exemple, en évaluant le comportement d'évanouissement sommaire de la fluorescence dans le système stratifié (1) à l'aide d'une fonction de modèle à ajuster aux mesures, est **caractérisé en ce que** les moments d'apparition des fluorescences dans les couches individuelles (2) du système stratifié (1) sont déterminés pour évaluer le comportement d'évanouissement sommaire de la fluorescence à résolution dans le temps du système stratifié (1) en considérant pour les fluorescences respectives des paramètres spécifiques aux couches en fonction du temps dans la fonction de modèle pour l'approximation à l'écoulement mesuré du comportement d'évanouissement sommaire, qui indiquent respectivement le début temporel de la fluorescence dans la couche concernée (2).

2. Procédé suivant la revendication 1 est **caractérisé en ce que** l'écart local géométrique entre les lieux d'apparition des fluorescences est respectivement calculé sur la base de la différence entre les paramètres spécifiques aux couches en fonction du temps par multiplication avec la vitesse de propagation de la lumière dans la couche respective du système stratifié.

3. Procédé suivant la revendication 2 est **caractérisé en ce que** les structures géométriques des couches fluorescentes du système stratifié sont calculées sur la base des écarts locaux entre les lieux d'apparition individuels de la fluorescence.

4. Procédé suivant la revendication 1 est **caractérisé en ce que** les paramètres spécifiques aux couches en fonction du temps, qui représentent les moments d'apparition des fluorescences individuelles du système stratifié, sont respectivement introduits comme paramètre de fit $tc_i$ dans la fonction de modèle appelée pour l'évaluation afin de calculer le comportement d'évanouissement sommaire de la fluorescence dans le système stratifié, par exemple pour un fit multiexponentiel suivant la formule générale (2)

$$\frac{I(t)}{I_0} = \sum_{i=1}^{p} \alpha_i \cdot e^{-\frac{t-tc_i}{\tau_i}} + b \quad (2) \quad \text{avec} \quad e^{-\frac{t-tc_i}{\tau_i}} = 0 \quad \text{pour} \quad t < tc_i$$

et

$I_o$: Intensité de fluorescence maximale
$I(t)$: Fluorescence au moment t
$\alpha_i$: Facteur préexponentiel i
$\tau_i$: Temps d'évanouissement du composant i dans couche i
$tc_i$: Moment de l'apparition de la fluorescence dans couche i
b: Intensité du fond.

Fig. 1

$$\frac{I(t)}{I_0} = \sum_{i=1}^{p} \alpha_i \cdot e^{-\frac{t-tc_i}{\tau_i}} + b$$

mit $e^{-\frac{t-tc_i}{\tau_i}} = 0$ für $t < tc_i$

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• WO 2006009910 A1


**In der Beschreibung aufgeführte Nicht-Patentliteratur**

• **K. KÖNIG ; I. RIEMANN.** High-resolution multiphoton tomography of human skin with subcellular spatial resolution and picosecond time resolution. *Journal of Biomedical Optics,* 2003, vol. 8 (3), 432-439
• **W. DENK ; J. H. STRICKLER.** W. W. Webb: Two-photon laser scanning microscopy. *Science,* 1990, vol. 248, 73-76
• **B. R. MASTERS ; P. T. C. SO ; E. GRATTON.** Multiphoton excitation fluorescence microscopy of in vivo human skin. *Ann. N.Y. Acad. Sci.,* 1998, vol. 838, 58-67
• **A. VON RÜCKMANN ; F. W. FITZKE ; A. C. BIRD.** Distribution of fundus autofluorescence with a scanning laser ophthalmoscope. *Br. J Ophthalmol,* 1995, vol. 79, 407-412
• **F. G. HOLZ et al.** Fundus autofluorescence and development of geographic atrophy in agerelated macular degeneration. *Invest Ophthalmol Vis Sci,* 2001, vol. 42, 1051-1056

• **F. C. DELORI et al.** vivo fluorescence of the ocular fundus exhibits retinal pigment epithelium lipofuscin characteristics. *Invest Ophthalmol Vis Sci,* 1995, vol. 36, 718-729
• **D. SCHWEITZER et al.** Die altersabhängige Makulopathie - Vergleichende Untersuchungen zwischen Patienten, deren Kindern und Augengesunden. *Ophthalmologe,* 2000, vol. 97, 84-90
• **D. SCHWEITZER et al.** In vivo measurement of time-resolved autofluorescence at the human fundus. *J Biomed Opt,* 2004, vol. 9, 1214-1222
• **D. SCHWEITZER et al.** Towards metabolic mapping of the human retina. *Microscopy Research and Technique,* 2007, vol. 70, 410-419
• **D. SCHWEITZER et al.** In vivo measurement of time-resolved autofluorescence at the human fundus. *J Biomed Opt,* 2004, vol. 9, 1214-1222
• *Absätze,* vol. 71, 109-111